# EUROPEAN PATENT APPLICATION

(11) **EP 3 332 722 A1**
(43) Date of publication of application: **13.06.2018**
(21) Application number: 15900301.1
(22) Date of filing: 06.08.2015
(51) Int. Cl.: A61B 17/80

(54) **DIVERGENT SEGMENTED BIPLANAR PLATE FOR BONE FIXATION**

(71) Applicant: Industrias Medicas Sampedro S.A.S., Sabaneta, Antioquia (CO)
(72) Inventor: TORO RESTREPO, Mauricio, Sabaneta Antioquia (CO); GOMEZ RAMIREZ, Jairo Fernando, Bogota (CO); VILLA MORENO, Daniela, Sabaneta Antioquia (CO); RIOS MENESES, Tatiana, Sabaneta Antioquia (CO)
(74) Representative: Martin Santos, Victoria Sofia
(86) International application number: PCT/IB2015/055979
(87) International publication number: WO 2017/021761

(57) **Abstract**

The present invention relates to a bone fixation plate, which is based on a divergent, biplanar and segmental (DBS) concept which consists in the use or self-stable screws with a divergent biplanar arrangement by segments, wherein each segment is composed by self-stable screws which diverge certain degrees to each other, ending thereby located on different fixation planes. Thus, the divergent biplanar fixation favors the stability in relation to torsional loads in locked systems, since unlike the fixation with cortical screws where the bone helps to support the loads by having an implant-bone contact, when self-stable screws are used the loads are transmitted in the interface between them and the implant, being very prone to "cutting out" and the fracture of the screws when these are located in only one fixation plane.

## Description

### TECHNICAL FIELD

The present invention relates to a concept of bone fixation, which is based on the divergent, biplanar and segmental (DBS) fixation of screws which consists in the use or self-stable screws with a divergent biplanar arrangement by segments, wherein each segment is composed by self-stable screws which diverge certain degrees to each other, ending thereby located on different fixation planes. In the conventional self-stable fixation, the loads are transmitted in the interface between the screws and the plate, these being propense to cutting out and fracture, wherein such situation is mitigated when the fixation is biplanar and also divergent, which is favorable for the stability in relation to torsion loads.

In the same manner, the DBS concept for osteosynthesis systems is indicated for fixing diaphyseal fractures, mainly in zones subjected to torsional stress wherein the fixation in only one plane is unstable, and where its use is also recommended in patients suffering from osteoporosis or low bone density, since it provides greater fixation stability.

### BACKGROUND OF THE INVENTION

When a fracture occurs or the fixation of bone fragments in a patient is required, it is necessary the use of fixation systems, among which are the plates, these elements allow to fix the bone through the insertion of a series of screws, which stabilize the bone fragments and allow the recovery thereof.

Thus, actually, most of the conventional bone fixation plates have a series of screws which are located on a same plane so as to enable the desired adjustment. However, it has been seen that this kind of fixation can be unstable.

In this regard, with the use of the conventional locking systems, several complications have been detected such as the damage of the screws or the loss of fixation, namely in zones subjected to torsion stress, wherein a biplanar fixation of the screws can help to avoid the loss of resistance caused by a fixation of the screws in just one plane.

Since the appearance of the osteosynthesis systems, there has been a continuous evolution and innovation by which the bone consolidation and the initial stabilization of fracture have been strengthened. The first solutions to appear were the locked systems (LCP) by the end of the 90s which although feature good results in the metaphyseal fixation, a large amount of failures at the diaphyseal level have been reported due to its low resistance to torsion loads¹, up to 69% lower than the conventional dynamic compression systems (DCP) in bones with osteoporosis², and due to problems that go from the fracture of the screws to the cutting out.
¹ Denard PJ, Doornink J, Phelan D, Madey SM, Fitzpatrick DC, Bottlang M, et al. Biplanar fixation of a locking plate in the diaphysis improves construct strength. "Clin Biomech (Bristol 2011;26(5):484-490.
² Fitzpatrick DC, Doornink J, Madey SM, Bottlang M. Relative stability of conventional and locked plating fixation in a model of the osteoporotic femoral diaphysis. "Clin Biomech (Bristol 2009;24(2):203-209.

Thus, the studies made in the state of the art about the failures of the locked systems, have reported causes of direct and indirect relation with the use of these which are³: ignorance about the surgical technique, change of angle during the insertion of the screws, lack of use of torque limiting screwdrivers, misalignment with the joint, not performing an adequate reduction, immediate support of the fractured limb.
³ Tan SLE, Balogh ZJ. Indications and limitations of locked plating. Injury 2009;40(7):683-691.

Thus, in searching for a solution to these problems, a large amount of studies have been developed in which an appropriate distribution and amount of the fixation screws⁴ has been tried to be established, leading to the developers of these systems to design and evaluate a large amount of new design concepts. An example of this, is the study developed by Patrick et al. in which the fixation in diaphyseal zones of large synthetic bones models have been evaluated using locked systems with convergent biplanar arrangement, the results obtained showed that such arrangement of screws improved the fixation strength to torsion stress both in bone with normal density and with low density.
⁴ Lee CH, Shih KS, Hsu CC, Cho T. Simulation-based particle swarm optimization and mechanical validation of screw position and number for the fixation stability of a femoral locking compression plate. Med Eng Phys 2014;36(1):57-64.

In this regard, there is a plurality of disclosures in the state of the art related to bone fixation plates, including the document US 2009228010 which refers to a bone fixation system having a bone plate and a specialized screws system, where the bone plate includes one or more bidirectional combination holes that can accommodate two bone screws in the same hole, wherein the screws are oriented in a non-parallel direction, such as divergent.

However, the plate described in this document features the disadvantage that it has only one hole where the screws are positioned, where there is not a two plane concept which allows to perform a more stable fixation.

On the other hand, document EP 2438870 discloses an orthopedic implant device for fixation of a fractured bone, comprising at least one opening extending from an upper surface to a lower surface, wherein the opening includes a first hole-portion and a second hole-portion, where the two hole-portions overlap each other at their ends to form a combination-hole.

However, this document also features the disadvantage of disclosing holes that create a certain angle to each other but which are located in the same plane, which is undesirable when patients with any type of condition or bone disease involving the quality of the bone tissue are treated.

There is another disclosure related to this type of plates which is found in document US 8192472 that shows a system for the internal fixation of a fractured bone including at least one bone plate, each bone plate having a plurality of holes and being configured to fit an anatomical surface of the fractured bone. The system also includes a plurality of fasteners including at least one locking fastener for attaching the bone plate to the bone, wherein at least one of the holes is a threaded hole.

However, this document establishes that the tilting between the screws is directly determined by the tilting of the plate but not by the angulation in each hole, which is undesirable since the plate must be bent or must have a flexible material and does not fulfill with its function in the most appropriate manner.

Finally, document US 2012158058 discloses a plating system having multiple and single locking mechanisms for general skeletal use, wherein the plating system allows for a pair of screws to be inserted into a bone in a crossed orientation and locked to the plate, wherein said system can be segmented and adjusted according to the needs defined directly by the surgeon.

According to the above, it is clear that there is a need in the state of the art to design and implement a bone fixation plate which is segmented and allows the insertion of screws such that an angle therebetween is formed, but wherein at the same time these screws are located in different planes, all of this in order to allow an adequate and desired fixation in order to be used in patients having any type of bone conditions, such as osteoporosis or alike.

### BRIEF DESCRIPTION OF THE DRAWINGS

Figure 1 corresponds to a perspective view of the bone fixation plate of the present invention composed by several segments.
Figure 2 corresponds to an upper plane view of the bone fixation plate of figure 1.
Figure 3 corresponds to a lower plane view of the bone fixation plate of figure 1.
Figure 4 corresponds to a cross-sectional view of a segment of the bone fixation plate of figure 1.

### DETAILED DESCRIPTION OF THE INVENTION

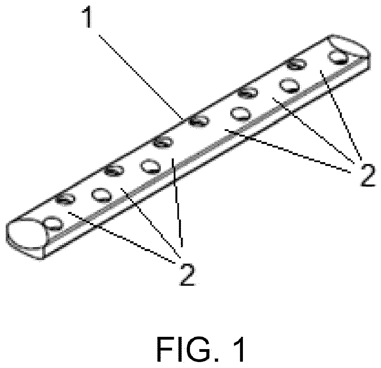

The bone fixation plate (1) of the present invention is composed by a plurality of segments (2), wherein each segment (2) is joined to each other by its ends and can be positioned according to the needs of the patient, such as shown in figure 1, wherein said segments can be located in an opposite manner.

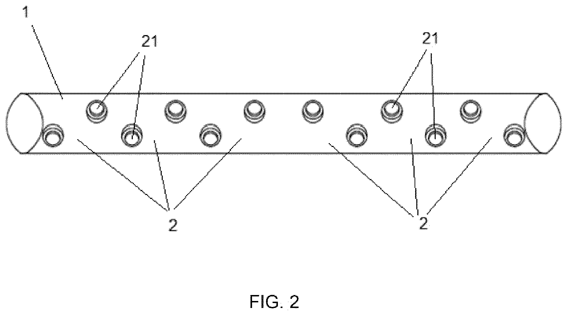

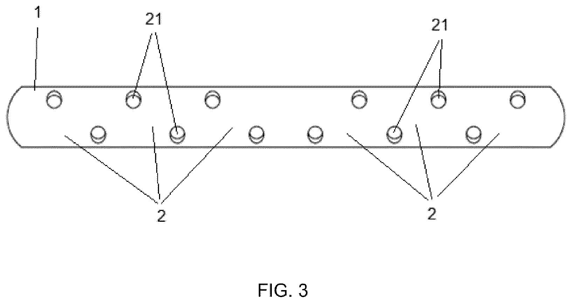

Thus, each of said segments (2) has two holes (21) which are located in an offset manner to each other, as can be seen in figures 2 and 3, i.e. the upper hole (21) can be located more forward or backward from the lower hole (21), depending on how the installation of the plate (1) is made. This offset of holes (21) is both vertically and horizontally, when an upper plane view of the plate is made, as shown in figure 2.

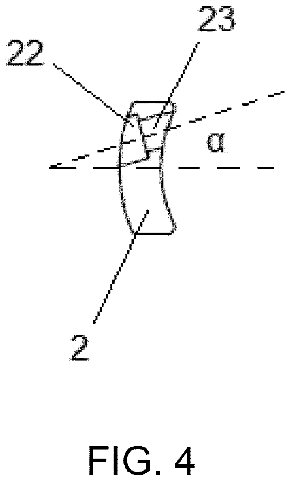

Similarly, these holes (21) have a tilting angle (α) with the vertical, which is less than or equal to 15° and allows to perform the insertion of the screws in the bone in a tilted manner for a stable fixation. Moreover, these holes (21) are composed by an upper portion (22) and a lower portion (23), wherein the upper portion (22) has a greater diameter than the lower portion (23), such as shown in figure 4, in order to assure that the head of the bone fixation screw is entirely located inside such upper portion (22) and is not visible or protrudes from the plate when the procedure is performed in the patient.

In a preferred embodiment of the invention, the plate (1) is made in a resistant material, such as biocompatible stainless steel or titanium.

## Claims

1. A bone fixation plate (1), **characterized by** being composed by two or more segments (2), wherein each segment (2) is joined to the next segment (2) by its ends, wherein each segment (2) comprises two holes (21) located in an offset manner to each other both vertically and horizontally, thereby forming an upper hole (21) and a lower hole (21), wherein these holes (21) have a tilting angle (α) with the vertical.

2. The plate (1) according to claim 1, **characterized by** having the tilting angle (α) between 5° and 15°.

3. The plate (1) according to claim 1 or 2, **characterized by** having holes (21) that are composed by an upper portion (22) and a lower portion (23), wherein the upper portion (22) has a greater diameter than the lower portion (23).

4. The plate (1) according to any preceding claim, **characterized by** being made in a resistant material, such as biocompatible stainless steel or titanium.
